(19) **Europäisches Patentamt**
**European Patent Office**
**Office européen des brevets**

(11) **EP 2 193 798 A1**

(12) **EUROPEAN PATENT APPLICATION**
published in accordance with Art. 153(4) EPC

(43) Date of publication:
09.06.2010 Bulletin 2010/23

(51) Int Cl.:
*A61K 38/00* (2006.01)     *A61K 9/107* (2006.01)
*A61K 47/10* (2006.01)     *A61K 47/12* (2006.01)
*A61P 17/00* (2006.01)     *A61P 17/06* (2006.01)

(21) Application number: 07806996.0

(22) Date of filing: 10.09.2007

(86) International application number:
PCT/JP2007/067564

(87) International publication number:
WO 2009/034604 (19.03.2009 Gazette 2009/12)

(84) Designated Contracting States:
AT BE BG CH CY CZ DE DK EE ES FI FR GB GR HU IE IS IT LI LT LU LV MC MT NL PL PT RO SE SI SK TR
Designated Extension States:
AL BA HR MK RS

(71) Applicant: Maruho Co., Ltd.
Osaka 531-0071 (JP)

(72) Inventors:
• AKAMATSU, Ryo
Kyoto-shi
Kyoto 600-8815 (JP)

• FUJII, Masahiro
Kyoto-shi
Kyoto 600-8815 (JP)
• SAKAGUCHI, Tomoki
Kyoto-shi
Kyoto 600-8815 (JP)
• HORISAWA, Eijiro
Kyoto-shi
Kyoto 600-8815 (JP)

(74) Representative: Riepe, Hans-Gerd et al
Pfenning, Meinig & Partner GbR
Patent- und Rechtsanwälte
Theresienhöhe 13
D-80339 München (DE)

(54) **LIQUID CRYSTAL EMULSION-TYPE PHARMACEUTICAL COMPOSITION CONTAINING CYCLOSPORINE, AND METHOD OF TREATING CUTANEOUS DISEASE THEREWITH**

(57) A dermal external pharmaceutical composition that excels in feeling at application or after application and that by enhancing of the transdermal absorption of cyclosporine, exerts medicinal benefits at low concentration. There is provided a liquid crystal emulsion-type pharmaceutical composition comprising cyclosporine, a hydrophilic nonionic surfactant, a lipophilic nonionic surfactant, an oil, a fatty acid that is insoluble in the oil at room temperature, a solid fatty alcohol that is insoluble in the oil at room temperature and a water-soluble polyhydric alcohol that is immiscible with the oil at room temperature, and a method of treating cutaneous diseases with the use of the pharmaceutical composition.

Fig. 1

EP 2 193 798 A1

**Description**

TECHNICAL FIELD

The present invention is related to a transdermal pharmaceutical composition that has superior skin absorbability of cyclosporine and much therapeutic efficacy on various cutaneous diseases such as allergic diseases and autoimmune diseases even at a low cyclosporine concentration, and a therapeutic method for cutaneous disease by topically administering the transdermal pharmaceutical composition

BACKGROUND ART

Cyclosporine is administrated by oral use and injection for the suppression of rejection responses in the transplantation of organs such as kidney, liver, heart, lung and pancreas; for the suppression of rejection responses in bone marrow transplantation and of graft-versus-host disease; and for Behcet's disease, severe aplastic anemia, and nephrotic syndrome. Cyclosporine is also administrated orally for severe psoriasis.

It is also known that cyclosporine is effective on various cutaneous diseases such as allergic diseases or autoimmune diseases such as atopic dermatitis.

The efficacy of Cyclosporine is much superior as described above. However, administration of Cyclosporine by oral use and injection have very high frequencies of the occurrence of systemic side effects such as renal function impairment, and cyclosporine frequently have many kinds of interactions with other medicines, so that there have been cases where administration must be limited.

In regard to various cutaneous diseases such as allergic diseases and autoimmune diseases, such as psoriasis and atopic dermatitis, the therapeutic effects can be achieved by directly applying a cyclosporine to the skin, which is the diseased site. As such, by topical treatment, an enhancement of the efficacy as a result of the increase in the drug concentration at the diseased site, and the reduction of the frequency of systemic side effects can be expected.

Under such circumstances, a treatment method involving direct application of cyclosporine preparation to a diseased site such as psoriasis or atopic dermatitis and proceeding transdermal absorption of cyclosporine has been attempted, and development of an effective external preparation with less side effects such as described above is desired. However, because the molecular weight of cyclosporine is very high and up to over 1200, it has been difficult to induce transdermal absorption of cyclosporine in general external preparations, and it has been difficult to make the efficacy to be manifested at a diseased site.

As an attempt to induce transdermal absorption of cyclosporine, a preparation for external use that contains cyclosporine at a high concentration is available. That is, there have been proposed a medicinal preparation for external use formed from an organic solvent for dissolving cyclosporine, a fatty acid ester of monohydric alcohol having 8 or more carbon atoms in total, which is in the liquid state at 25°C, and/or an alkanolamine which is in the liquid state at 25°C, an oily base which is in the solid state at 25°C, and a surfactant (Patent Document 1); a external preparation containing an organic liquid, a solid oily base, and a surfactant (Patent Document 2); an oil-in-water type emulsion composition containing a polycarboxylic acid polyalkyl ester which is liquid at normal temperature, an oil having an I.O.B of 0 to 0.25, which is liquid at normal temperature, and a surfactant (Patent Document 3); a gel or ointment preparation containing oleyl alcohol, with propylene glycol as a main base (Patent Document 4); a spontaneously emulsified emulsion (Patent Document 5); and a pharmaceutical preparation in a colloidal form (Patent Document 6). Since these external preparations contain cyclosporine at a high concentration, they are preparations having not only the adverse side effects derived from cyclosporine, but also bad texture because of the high mixing ratio of an oily component or water-soluble polyhydric alcohol, and skin irritancy derived from the high mixing ratio of a surfactant.

Meanwhile, an ointment preparation having the content of cyclosporine lowered by applying lauroylsarcosine as a transdermal absorption enhancer, has been proposed (Patent Document 7). However, since the preparation is prepared using an ointment base or an oil as a base at a very high mixing ratio, the preparation gives a poor feeling of use.

In a transdermal pharmaceutical composition, since having a good texture during spreading and after application is one of important factors, none of these external preparations are quite satisfactory as pharmaceutical products.

Patent Document 1: JP-A No. 5-310591 (WO 93/00160)
Patent Document 2: JP-A No. 7-188046
Patent Document 3: JP-A No. 7-278007 (WO 95/22343)
Patent Document 4: JP-A No. 8-133979
Patent Document 5: JP-A No. 10-7584 (EP 0793966)
Patent Document 6: JP-W No. 2005-516931 (WO 03/051385)
Patent Document 7: JP-A No. 7-25784

## DISCLOSURE OF THE INVENTION

### Problems to be Solved by the Invention

It is an object of the present invention to provide a transdermal pharmaceutical composition, in which the efficacy is exhibited with cyclosporine at a low concentration by increasing the transdermal absorbability of cyclosporine. It is another object to provide a transdermal pharmaceutical composition having an excellent texture during spreading and after application. It is still another object to provide a method of treating a cutaneous disease by topically administering the transdermal pharmaceutical composition.

### Means for Solving the Problems

The inventors of the present invention devotedly conducted researches on the appropriate chemical composition for a transdermal pharmaceutical composition containing cyclosporine, and as a result, they succeeded in obtaining the chemical composition of a transdermal pharmaceutical composition with sufficient efficacy in spite of a small content of cyclosporine, by manufacturing a liquid crystal emulsion type pharmaceutical composition.

Furthermore, the inventors also succeeded in obtaining a transdermal pharmaceutical composition which has a good texture during spreading and after application even though cyclosporine is contained, by making the transdermal pharmaceutical composition into a liquid crystal emulsion type pharmaceutical composition.

That is, the present invention provides a liquid crystal emulsion type pharmaceutical composition containing cyclosporine and a nonionic surfactant, and a method of treating a cutaneous disease in a mammal, the method including topically administering a therapeutically effective amount of the liquid crystal emulsion type pharmaceutical composition to the mammal suffering from the cutaneous disease.

Furthermore, it is preferable for the pharmaceutical composition to contain, as an oil phase component constituting the liquid crystal emulsion type pharmaceutical composition of the present invention, an oil, a fatty acid that is insoluble in the oil, and a solid fatty alcohol that is insoluble in the oil.

It is also preferable for the pharmaceutical composition to contain, as an aqueous phase component constituting the liquid crystal emulsion type pharmaceutical composition of the present invention, a water-soluble polyhydric alcohol that is immiscible with the oil.

### EFFECT OF THE INVENTION

According to the present invention, there is provided a transdermal pharmaceutical composition which can sufficiently exhibit the efficacy in spite of a small content of cyclosporine. There is also provided a transdermal pharmaceutical composition having an excellent texture during spreading and after application.

### BRIEF DESCRIPTIONS OF THE DRAWINGS

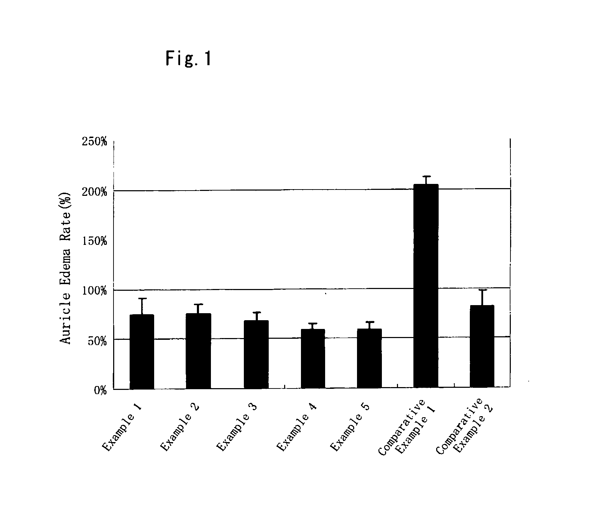

Fig. 1 is a diagram showing the results of a pharmacological test.

### BEST MODE FOR CARRYING OUT THE INVENTION

The liquid crystal emulsion type pharmaceutical composition of the present invention is characterized by the constitution of oil phase components. That is, among the constituent components, it is required that the fatty acid and the solid fatty alcohol are insoluble in a liquid oil component, which is an oil. Therefore, it is desirable that the polarity of the liquid oil component is relatively low. If the polarity of the liquid oil component is increased, the fatty acid and the solid fatty alcohol are uniformly dissolved, and it is difficult for the components to form a liquid crystal structure.

When a constitution in which the fatty acid and the solid fatty alcohol are insoluble in the liquid oil component, which is an oil, is adopted, it becomes easier for the fatty acid and the solid fatty alcohol, together with a nonionic surfactant, to form an oriented state near the interface. If a specific zwitterionic amino acid is added to this state, further stabilization of the interface is attempted, and the occurrence in which the liquid crystal emulsion type pharmaceutical composition maintains a more stable state in terms of formulation, is made possible.

According to the present invention, the liquid crystal emulsion type pharmaceutical composition refers to a composition in which, when observed with a microscope (object lens: 40 times magnification, ocular lens: 10 times magnification), distinct emulsion particles are not recognized, and when observed with a microscope (object lens: 4 to 40 times magnification, ocular lens: 10 times magnification) equipped with a simple polarizing filter, refracted light resulting from optical anisotropy is recognized.

Cyclosporine that is included as an active pharmaceutical ingredient is a known compound, and cyclosporine A, cyclosporine B, cyclosporine C, cyclosporine D and cyclosporine H are known. All of the compounds can be used in the present invention, but cyclosporine A is preferred. These cyclosporine compounds can be purchased as reagents from Alexis, MP Biomedicals, Inc., LKT Labs, Inc., Toronto Research Chemicals, Inc. and the like. The amount of cyclosporine contained in the pharmaceutical composition of the present invention is not particularly limited as long as it is an amount effective in the treatment of the cutaneous disease to which cyclosporine is applied, but the content is 0.01 to 1% by weight, preferably 0.05 to 0.75% by weight, and more preferably 0.05 to 0.5% by weight, relative to the total amount of the pharmaceutical composition obtainable by the present invention.

The surfactant that can be used in the present invention is a nonionic surfactant, and it is preferable to use a hydrophilic nonionic surfactant and a lipophilic nonionic surfactant in combination.

The lipophilic nonionic surfactant is preferably a surfactant having an HLB value of 2.0 to 7.0, and specific examples thereof include glycerin monostearate, sorbitan monostearate, diglyceryl monooleate, diglyceryl monostearate, tetraglyceryl monostearate, polyoxyethylene behenyl ether, and the like. Preferred examples include glycerin monostearate, diglyceryl monostearate, and tetraglyceryl monostearate, and more preferred examples include glycerin monosterate and tetraglyceryl monostearate. These can be used singly or in combination of two or more kinds, and the amount of incorporation is 0.1 to 5% by weight, and preferably 0.5 to 4% by weight, relative to the total amount of the pharmaceutical composition obtainable by the present invention. If the amount of incorporation is less than 0.1% by weight, the liquid crystal structure cannot be maintained, and if the amount exceeds 5% by weight, skin irritancy is prone to occur.

The hydrophilic nonionic surfactant is preferably a surfactant having an HLB value of 10.0 to 18.0, and specific examples thereof include polyoxyethylene (20) polyoxypropylene (4) cetyl ether, polyoxyethylene cetyl ether, polyoxyl 25 stearate, polyoxyethylene hardened castor oil 60, and the like. Preferred examples include polyoxyethylene (20) polyoxypropylene (4) cetyl ether, and polyoxyethylene hardened castor oil 60. These can be used singly or in combination of two or more kinds, and the amount of incorporation is 0.1 to 5% by weight, and preferably 0.5 to 4% by weight, relative to the total amount of the pharmaceutical composition obtainable by the present invention. If the amount of incorporation is less than 0.1% by weight, the liquid crystal structure cannot be maintained, and if the amount exceeds 5% by weight, skin irritancy is prone to occur.

The oil phase components that can be used in the present invention are not particularly limited as long as they are components generally used in external preparations and cosmetics. However, it is preferable to use a combination of at least an oil, a fatty acid that is insoluble in the oil, and a solid fatty alcohol that is insoluble in the oil.

Examples of the oil that can be used in the present invention include hydrocarbons, fatty acid esters, triglycerides, and liquid fatty alcohols. Examples of the hydrocarbons include squalane, liquid paraffins and the like, and examples of the fatty acid esters include octyl dodecyl myristate, isopropyl myristate, diethyl sebacate and the like. Examples of the triglycerides include glycerin triisooctanoate, medium-chain fatty acid triglycerides and the like. The liquid fatty alcohols refer to fatty alcohols which are liquid at room temperature, and specific examples thereof include octyldodecanol, hexyldecanol and the like, but preferred examples include squalane, glycerin triisooctanoate, and octyldodecanol. If an oil that dissolves a fatty acid and a solid fatty alcohol is used, it is difficult to maintain the liquid crystal structure. Therefore, it is necessary to select an oil that does not dissolve a fatty acid and a solid fatty alcohol at room temperature. These can be used singly or in combination of two or more kinds, and the amount of incorporation thereof is 0.5 to 10% by weight, and preferably 1 to 9% by weight, relative to the total amount of the pharmaceutical composition obtainable by the present invention.

The phrase "is/are insoluble in an/the oil" according to the present invention means that the fatty acid or the solid fatty alcohol is insoluble in the desired oil at room temperature, or that as for the desired amount of incorporation, since the fatty acid or the solid fatty alcohol is present in an amount equal to or greater than the saturation solubility for the desired oil, the oil and the fatty acid or the solid fatty alcohol are not in a dissolved state.

The range room temperature according to the present invention refers to 1 to 30°C.

In the present invention, the fatty acid that is insoluble in the oil is preferably a fatty acid which is solid at room temperature, and more preferably a saturated, linear type fatty acid having 16 to 22 carbon atoms. Therefore, specific examples include palmitic acid, stearic acid, behenic acid and the like, and a preferred example is behenic acid. These can be used singly or in combination of two or more kinds, and the amount of incorporation is 0.1 to 1% by weight relative to the total amount of the pharmaceutical composition obtainable by the present invention.

In the present invention, the solid fatty alcohol that is insoluble in the oil refers to a fatty alcohol which is solid at room temperature. Specifically, a saturated, linear type solid fatty alcohol having 16 to 22 carbon atoms is preferred, and examples thereof include cetanol, cetostearyl alcohol, stearyl alcohol, behenyl alcohol and the like, while preferred examples include cetostearyl alcohol and behenyl alcohol. These can be used singly or in combination of two or more kinds, and the amount of incorporation is 0.1 to 10% by weight, and preferably 0.5 to 6% by weight, relative to the total amount of the pharmaceutical composition obtainable by the present invention.

The aqueous phase components that can be used in the present invention are not particularly limited as long as they are components generally used in external preparations and cosmetics. However, it is preferable to use at least a water-soluble polyhydric alcohol that is immiscible with the oil.

In the present invention, examples of the water-soluble polyhydric alcohol that is immiscible with the oil include 1,3-butylene glycol, glycerin, propylene glycol, dipropylene glycol and the like, and preferred examples include 1,3-butylene glycol and glycerin. These can be used singly or in combination of two or more kinds, and the amount of incorporation is 1 to 50% by weight, and preferably 5 to 50% by weight, relative to the total amount of the pharmaceutical composition obtainable by the present invention.

The phrase "immiscible with an/the oil" according to the present invention means that the water-soluble polyhydric alcohol is not in a state of being miscible with the oil used, at room temperature.

An emulsion stabilizer can also be incorporated for the stabilization of the liquid crystal structure. Examples of the emulsion stabilizer include L-arginine, glycine, sodium N-acyl-L-glutamate, and the like, and L-arginine is preferred. These can be used singly or in combination of two or more kinds, and the amount of incorporation is 0.01 to 5% by weight, and preferably 0.05 to 0.5% by weight, relative to the total amount of the pharmaceutical composition obtainable by the present invention.

Since the pharmaceutical composition of the present invention has structural viscosity, it is not necessary to incorporate a thickening agent or the like, but if necessary, the pharmaceutical composition can be prepared to have a desired viscosity by incorporating a thickening agent such as a water-soluble polymer such as a carboxyvinyl polymer, or a clay mineral such as bentonite.

Furthermore, if necessary, a pH adjusting agent such as citric acid or sodium citrate, a colorant, a fragrance, a pigment, a preservative, an antioxidant, a stabilizer, an ultraviolet absorbent and the like can be appropriately incorporated in the amounts in the range constituting the purpose of the present invention.

In the pharmaceutical composition of the present invention, cyclosporine is present in the state of being dissolved in a solubilizer. Any solubilizer for cyclosporine can be used, irrespective of being an oil phase component or an aqueous phase component, as long as it is a solubilizer that can dissolve the required amount of cyclosporine. However, a solubilizer in which $\alpha$ = 10 to 75 ($\alpha$ means tan $\alpha$, which represents the balance of organicity and inorganicity) is preferred, and hexyldecanol, octyldodecanol and 1,3-butylene glycol are more preferred.

According to a preferred embodiment, the liquid crystal emulsion type pharmaceutical composition of the present invention contains 0.01 to 1% by weight of cyclosporine, 0.1 to 5% by weight of a hydrophilic nonionic surfactant, 0.1 to 5% by weight of a lipophilic nonionic surfactant, 0.5 to 10% by weight of an oil, 0.1 to 1% by weight of a fatty acid that is insoluble in the oil, 0.1 to 6% by weight of a solid fatty alcohol that is insoluble in the oil, and 1 to 50% by weight of a water-soluble polyhydric alcohol that is immiscible with the oil, based on the total weight of the composition.

According to a more preferred embodiment, the liquid crystal emulsion type pharmaceutical composition of the present invention contains 0.05 to 1% by weight of cyclosporine, 0.5 to 5% by weight of glycerin monostearate, 0.5 to 5% by weight of polyoxyethylene hardened castor oil 60, 1 to 10% by weight of squalane, 0.1 to 1% by weight of behenic acid, 0.5 to 6% by weight of cetostearyl alcohol, and 1 to 50% by weight of glycerin and/or 1,3-butylene glycol, based on the total weight of the composition.

The method of manufacturing the liquid crystal emulsion type pharmaceutical composition of the present invention is not particularly limited, but a desired liquid crystal emulsion type pharmaceutical composition can be prepared by a process of preparing an oil phase containing an oil, a fatty acid that is insoluble in the oil, a solid fatty alcohol that is insoluble in the oil, a lipophilic nonionic surfactant, a hydrophilic nonionic surfactant and the like, and an aqueous phase containing a water-soluble polyhydric alcohol that is immiscible with the oil, an amino acid, a pH adjusting agent, water and the like, and emulsifying the phases by a method of adding the aqueous phase into the oil phase. Furthermore, in regard to cyclosporine, use is made of a product which has been previously dissolved in the oil or the water-soluble polyhydric alcohol that is immiscible with the oil. Upon performing emulsification, the aqueous phase may be used such that the total amount is used all at once, or a portion of the aqueous phase may be used to emulsify and to thereby form a concentrated emulsion (conc. base), and then the rest of the aqueous phase may be divided into several portions and mixed with the conc. base to dilute it. When the aqueous phase is divided into several portions, the aqueous phase components may all be dissolved, and then the aqueous phase may be divided and used to emulsify. Alternatively, aqueous phase components of different compositions may be used at the conc. base production stage and at the dilution stage, for example, by adding the amino acid to the aqueous phase only at the time of manufacturing the conc. base.

The conditions used at the time of preparing the oil phase of the present invention are appropriately selected, but in order to uniformly mix and dissolve the oil, the fatty acid that is insoluble in the oil, the solid fatty alcohol that is insoluble in the oil, the lipophilic nonionic surfactant and the hydrophilic nonionic surfactant, it is preferable to mix and dissolve the various components at 60 to 90°C, and preferably 65 to 85°C. If the temperature is excessively low, it may become difficult to uniformly mix and dissolve the oil phase. If the temperature is excessively high, there is a fear that the various components may be altered.

Furthermore, the conditions used at the time of preparing the aqueous phase of the present invention may be appropriately selected, but in order to uniformly mix and dissolve the aqueous phase components that are appropriately selected from the water-soluble polyhydric alcohol that is immiscible with the oil, the amino acid, the pH adjusting agent, the preservative, the stabilizer and the like in water, it is preferable to mix and dissolve these components at room temperature to 90°C, and preferably room temperature to 80°C.

Subsequently, the preparation method of the concentrated emulsion (conc. base) involves slowly adding the aqueous phase to the oil phase while subjecting the oil phase to stirring. The temperature at the time of preparing the conc. base is appropriately selected based on the type of the oil phase and the aqueous phase, or the like, but the temperature is usually 50°C to 90°C at the initiation of stirring, and room temperature at the completion of stirring. If the temperature is excessively low, emulsification may become difficult.

The conditions used at the time of preparing the aqueous phase for the second step of the present invention are appropriately selected, but the preservative, the pH adjusting agent and the stabilizer are dissolved in water. In order to uniformly mix and dissolve these components, it is preferable to mix and dissolve them at room temperature to 90°C, and preferably room temperature to 80°C. When this aqueous phase and the conc. base are uniformly mixed at room temperature, and the mixture is further subjected to stirring and degassing, the desired liquid crystal emulsion type pharmaceutical composition can be obtained. Upon the addition of the aqueous phase, warming is not necessary, and the step can be carried out at room temperature.

The pharmaceutical composition of the present invention can be prepared using an emulsifying machine that is used for preparing conventional transdermal pharmaceutical preparations but may also be prepared using a strong stirring machine such as a Manton-Gaulin emulsifier or a microfluidizer, as necessary.

In regard to the administration of the pharmaceutical composition of the present invention, the pharmaceutical composition can be directly spread on the diseased site several times, for example, one to three times, a day, or the pharmaceutical composition can be converted into the form of a patch, a plaster or a cataplasm, and this can be similarly applied to the diseased site several times a day. The frequency of application can be appropriately increased or decreased according to the severity of the corresponding disease. The pharmaceutical composition of the present invention can be used in the treatment of cutaneous diseases for which cyclosporine is effective in the treatment thereof, such as atopic dermatitis and psoriasis. The subjects to which the pharmaceutical composition of the present invention is topically administered are mammals such as a human, a dog, a cat, a horse, a cattle and a monkey, and a preferred subject is a human.

Hereinafter, the present invention will be explained in more detail by way of examples and test examples, but the present invention is not intended to be limited to these.

EXAMPLES

[Example 1] Preparation of liquid crystal emulsion type composition

Squalane, behenic acid, cetostearyl alcohol, glycerin monostearate, and polyoxyethylene (20) polyoxypropylene (4) cetyl ether at the composition ratios shown in the following Table 1 were dissolved at 85°C (oil phase). Cyclosporine A was dissolved in 1,3-butylene glycol at 60°C (API[Active Pharmaceutical Ingredients] phase). Furthermore, a pH adjusting agent and L-arginine were dissolved in water at 85°C (aqueous phase). The API phase was added to the oil phase, and the aqueous phase was further added thereto. The mixture was stirred (conc. base).

Separately, 1, 3-butylene glycol and a pH adjusting agent were added to water at room temperature and were dissolved (late-added aqueous phase). The late-added aqueous phase was added to the conc. base at room temperature, and the mixture was subjected to stirring and degassing, to obtain the desired preparation.

[Example 2 to Example 10 and Comparative Example 1]

The same operation as in Example 1 was carried out, and thus preparations were prepared at the composition ratios shown in Table 1.

[Table 1]

| Name of component (% by weight) | Example 1 | Example 2 | Example 3 | Example 4 | Example 5 | Example 6 | Example 7 | Example 8 | Example 9 | Example 10 | Comparative Example 1 |
|---|---|---|---|---|---|---|---|---|---|---|---|
| Cyclosporine A | 0.1 | 0.25 | 0.5 | 0.75 | 1.0 | 0.2 | 0.2 | 0.2 | 0.2 | 0.2 | - |
| Squalane | 1.4 | 8.4 | 5.6 | 8.4 | 7.0 | 2.8 | - | 0.8 | 0.8 | 0.8 | 8.4 |
| Octyldodecanol | - | - | - | - | - | - | 1.4 | - | - | - | - |
| Glycerin triisooctanoate | - | - | - | - | - | - | - | 0.6 | 0.6 | 0.6 | - |
| Behenic acid | 0.2 | 0.9 | 0.6 | 0.9 | 0.8 | 0.3 | 0.2 | 0.2 | 0.2 | 0.2 | 0.9 |
| Cetostearyl alcohol | 0.9 | 5.1 | 3.4 | 5.1 | 4.3 | 1.7 | - | 0.9 | 0.9 | 0.9 | 5.1 |
| Behenyl alcohol | - | - | - | - | - | - | 0.9 | - | - | - | - |
| Glycerin monostearate | 0.6 | 3.6 | 2.4 | 3.6 | 3.0 | 1.2 | - | - | 0.6 | 0.6 | 3.6 |
| Tetraglyceryl monostearate | - | - | - | - | - | - | 0.6 | 0.6 | - | - | - |
| Polyoxyethylene (20) polyoxypropylene (4) cetyl ether | 0.6 | - | - | - | - | - | - | - | 0.6 | - | - |
| Polyoxyethylene -hardened castor oil | 60 | 3.6 | 2.4 | 3.6 | 3.0 | 1.2 | 0.6 | 0.6 | - | 0.6 | 3.6 |
| 1,3-Butylene glycol | 9.0 | 39.0 | 26.0 | 39.0 | 32.5 | 13.0 | 9.0 | 9.0 | 9.0 | - | 39.0 |
| Glycerin | - | 9.0 | 6.0 | 9.0 | 7.5 | 3.0 | - | - | - | 9.0 | 9.0 |
| L-arginine | 0.1 | 0.5 | 0.3 | 0.5 | 0.4 | 0.2 | 0.1 | 0.1 | - | 0.1 | 0.5 |
| Glycine | - | - | - | - | - | - | - | - | 0.1 | - | - |

(continued)

| Name of component (% by weight) | Example 1 | Example 2 | Example 3 | Example 4 | Example 5 | Example 6 | Example 7 | Example 8 | Example 9 | Example 10 | Comparative Example 1 |
|---|---|---|---|---|---|---|---|---|---|---|---|
| pH adjusting agent | Appro -priate amount | - | - | - | - | - | Appro -priate amount | Appro -priate amount | Appro -priate amount | Appro -priate amount | - |
| Purified water | Balance | Balance | Balance | Balance | Balance | Balance | Balance | Balance | Balance | Balance | Balance |

[Comparative Example 2]

Protopic (registered trademark) Ointment 0.1%; ointment preparation containing 1.02 mg of tacrolimus hydrate (manufactured by Astellas Pharma Inc.)

[Test Example 1] Emulsion state and emulsion particle size

Small amounts of the preparations of Example 1 to Example 10 were placed on slide glasses, cover glasses were placed thereon, and then the samples were subjected to microscopic observation (object lens: 40 times magnification, ocular lens: 10 times magnification). Furthermore, the presence or absence of optical anisotropy was also observed using a simple polarizing filter (object lens: 4 to 40 times magnification, ocular lens: 10 times magnification), according to necessity.

<Evaluation criteria>

A: Distinct emulsion particles are not recognized, and refracted light resulting from optical anisotropy is recognized.
B: Emulsion particles having a size of 1 $\mu$m or less only
C: Emulsion particles having a size of 1 to 5 $\mu$m only
D: Emulsion particles having a size of 1 to 5 $\mu$m are mainly present, and particles having a size of several to several ten $\mu$m are also included.
E: Others

[Table 2]

|  | Result |  | Result |
| --- | --- | --- | --- |
| Example 1 | A | Example 6 | A |
| Example 2 | A | Example 7 | A |
| Example 3 | A | Example 8 | A |
| Example 4 | A | Example 9 | A |
| Example 5 | A | Example 10 | A |

In all of the respective compositions of the examples, distinct particles were not recognized, and refracted light resulting from optical anisotropy was recognized. Thus, it was confirmed that the compositions were liquid crystal emulsion type compositions.

[Test Example 2] Formulation stability test

The preparation of Example 6 was stored for 3 weeks under the conditions indicated in Table 3, and the presence or absence of the precipitation of crystals was verified with a microscope (object lens: 40 times magnification, ocular lens: 10 times magnification), while the presence or absence of the separation of preparation was verified by visual inspection.
The results are presented in Table 3, and it was confirmed that regardless of the conditions for storing the preparation, the preparation is stable, without crystals being precipitated.

[Table 3]

|  | Storage condition | Result |
| --- | --- | --- |
| Precipitation of crystals | 5°C | None |
|  | 40°C | None |
| Separation of preparation | 5°C | None |
|  | 40°C | None |

[Test Example 3] API stability test

The content of cyclosporine A obtained when the preparation of Example 6 was stored for 3 weeks under the conditions indicated in Table 4, was measured. In order to measure the content of cyclosporine A, a tetradecanophenone solution and acetonitrile were added to about 0.6 g of the preparation, and the mixture was stirred and then centrifuged. A portion of the supernatant thus obtained was used as a sample solution. This was subjected to the measurement of the content of cyclosporine A contained in each sample by reverse phase high performance liquid chromatography (detection wavelength; 210 nm, mobile phase; water:acetonitrile mixed liquid = 2:3), and thus the residual rate (%) of cyclosporine A with respect to the initial value was determined.

The results are presented in Table 4, and it was confirmed that regardless of the conditions for storing the preparation, cyclosporine A in the preparation is stable.

[Table 4]

| Storage condition | Residual rate (%) |
|---|---|
| 5°C | 103.7 |
| 40°C | 99.9 |

[Test Example 4] Pharmacological test

A pharmacological test was carried out for the preparations of Example 1 to Example 5, Comparative Example 1 and Comparative Example 2. 0.1 mL of a 3% picryl chloride-ethanol solution was applied to the abdomen of a mouse which had been shaved at the abdomen (sensitization). After 6 days therefrom, 0.02 mL of a 1% picryl chloride-acetone solution was applied to the right ear to cause induction. One hour after the induction, each preparation (10 mg) was applied to the auricle of the right ear, and 24 hours after the induction, the right and left auricles were punched with a puncher. The weights of the punched auricles were measured, and the edema rate was calculated using the following calculation formula.

[Calculation Formula 1]

Edema rate = (weight of auricle of right ear - weight of auricle of left ear)/weight of auricle of left ear × 100 (%)

The results are presented in Fig. 1, and the results were that the respective compositions of the examples showed markedly low auricle edema rates as compared with the composition of Comparative Example 1, which was a placebo, and showed equal or slightly lower auricle edema rates as compared with the composition of Comparative Example 2, which was a commercially available preparation. Therefore, it can be seen that the respective compositions of the examples have excellent pharmacological actions.

[Test Example 5] Skin cumulative irritation test

The composition of Example 6 was subjected to a skin cumulative irritancy test using a male rabbit.

The preparation (100 mg) was applied to the normal skin (2.5 cm × 2.5 cm) of the rabbit, and after a lapse of 6 hours from the application, the preparation was wiped out with lukewarm water using absorbent cotton. About 23 hours after the application, the skin response was observed. This operation was carried out consecutively for 7 days. The average score for each day is presented in Table 5.

The evaluation of skin response was made according to the judgment criteria of Draize, J.H. (Appraisal of the safety of chemicals in foods, drugs and cosmetics, The Association of Food and Drug Officials of the United States, Topeka, Kansas, 46-59, 1965).

The results are presented in Table 5, and the preparation was judged as a weak irritant so that there is no problem in using the preparation as an external preparation

[Table 5]

| | Skin response | Average score for each day | | | | | | |
|---|---|---|---|---|---|---|---|---|
| | | 1 | 2 | 3 | 4 | 5 | 6 | 7 |
| Example 6 | Erythema and crust formation | 0.3 | 0.7 | 0.7 | 0.7 | 1.0 | 1.0 | 1.0 |
| | Edema | 0 | 0 | 0 | 0 | 0 | 0 | 0 |
| | Total | 0.3 | 0.7 | 0.7 | 0.7 | 1.0 | 1.0 | 1.0 |

**Claims**

1. A liquid crystal emulsion type pharmaceutical composition comprising cyclosporine and a nonionic surfactant.

2. The pharmaceutical composition according to claim 1,
   wherein the content of cyclosporine is 0.01 to 1% by weight.

3. The pharmaceutical composition according to claim 1 or 2, wherein a hydrophilic nonionic surfactant and a lipophilic nonionic surfactant are used in combination as the nonionic surfactant.

4. The pharmaceutical composition according to any one of claims 1 to 3, comprising an oil, a fatty acid that is insoluble in the oil, and a solid fatty alcohol that is insoluble in the oil, as oil phase components constituting the liquid crystal emulsion type pharmaceutical composition.

5. The pharmaceutical composition according to claim 4,
   wherein the fatty acid that is insoluble in the oil is a saturated, linear type fatty acid having 16 to 22 carbon atoms.

6. The pharmaceutical composition according to claim 4 or 5, wherein the solid fatty alcohol that is insoluble in the oil is a saturated, linear type alcohol having 16 to 22 carbon atoms.

7. The pharmaceutical composition according to any one of claims 1 to 6, comprising a water-soluble polyhydric alcohol that is immiscible with the oil, as an aqueous phase component constituting the liquid crystal emulsion type pharmaceutical composition.

8. The pharmaceutical composition according to claim 7,
   wherein the water-soluble polyhydric alcohol that is immiscible with the oil is selected from glycerin, propylene glycol, dipropylene glycol and 1,3-butylene glycol.

9. A method of treating a cutaneous disease in a mammal, comprising topically administering a therapeutically effective amount of the pharmaceutical composition according to claim 1 to the mammal suffering from the cutaneous disease.

10. The method according to claim 9, wherein the content of cyclosporine contained in the pharmaceutical composition is 0.01 to 1% by weight.

11. The method according to claim 9 or 10, wherein a hydrophilic nonionic surfactant and a lipophilic nonionic surfactant are used in combination as the nonionic surfactant contained in the pharmaceutical composition.

12. The method according to any one of claims 9 to 11,
    wherein the pharmaceutical composition contains an oil, a fatty acid that is insoluble in the oil, and a solid fatty alcohol that is insoluble in the oil, as oil phase components constituting the pharmaceutical composition.

13. The method according to claim 12, wherein the fatty acid that is insoluble in the oil contained in the pharmaceutical composition is a saturated, linear type fatty acid having 16 to 22 carbon atoms.

14. The method according to claim 12 or 13, wherein the solid fatty alcohol that is insoluble in the oil contained in the pharmaceutical composition is a saturated, linear type alcohol having 16 to 22 carbon atoms.

15. The method according to any one of claims 9 to 14,
wherein the pharmaceutical composition contains a water-soluble polyhydric alcohol that is immiscible with the oil, as an aqueous phase component constituting the pharmaceutical composition.

16. The method according to claim 15, wherein the water-soluble polyhydric alcohol that is immiscible with the oil contained in the pharmaceutical composition is selected from glycerin, propylene glycol, dipropylene glycol and 1,3-butylene glycol.

17. The method according to any one of claims 9 to 16,
wherein the cutaneous disease is selected from atopic dermatitis and psoriasis.

Fig. 1

## INTERNATIONAL SEARCH REPORT

| | International application No. |
|---|---|
| | PCT/JP2007/067564 |

A.   CLASSIFICATION OF SUBJECT MATTER
*A61K38/00*(2006.01)i, *A61K9/107*(2006.01)i, *A61K47/10*(2006.01)i, *A61K47/12* (2006.01)i, *A61P17/00*(2006.01)i, *A61P17/06*(2006.01)i

According to International Patent Classification (IPC) or to both national classification and IPC

B.   FIELDS SEARCHED

Minimum documentation searched (classification system followed by classification symbols)
A61K38/00, A61K9/107, A61K47/10, A61K47/12, A61P17/00, A61P17/06

Documentation searched other than minimum documentation to the extent that such documents are included in the fields searched
Jitsuyo Shinan Koho         1922-1996   Jitsuyo Shinan Toroku Koho   1996-2007
Kokai Jitsuyo Shinan Koho   1971-2007   Toroku Jitsuyo Shinan Koho   1994-2007

Electronic data base consulted during the international search (name of data base and, where practicable, search terms used)
CAplus(STN), JMEDPlus(JDream2), JST7580(JDream2), JSTPlus(JDream2)

C.   DOCUMENTS CONSIDERED TO BE RELEVANT

| Category* | Citation of document, with indication, where appropriate, of the relevant passages | Relevant to claim No. |
|---|---|---|
| E,X | JP 2007-269795 A  (Maruho Kabushiki Kaisha), 18 October, 2007 (18.10.07), (Family: none) | 1-8 |
| Y | JP 2005-516931 A  (Jagotec AG.), 09 June, 2005 (09.06.05), Particularly, Claims & EP 1455810 A1          & WO 2003/51385 A1 & US 2005/106189 A1 | 1-8 |
| Y | JP 7-188046 A  (LTT Institute Co., Ltd.), 25 July, 1995 (25.07.95), Particularly, Claims; examples (Family: none) | 1-8 |

☒   Further documents are listed in the continuation of Box C.          ☐   See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered   to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 12 November, 2007 (12.11.07) | 20 November, 2007 (20.11.07) |

| Name and mailing address of the ISA/ | Authorized officer |
|---|---|
| Japanese Patent Office | |
| Facsimile No. | Telephone No. |

Form PCT/ISA/210 (second sheet) (April 2007)

<div align="center">**INTERNATIONAL SEARCH REPORT**</div>

| International application No. |
| --- |
| PCT/JP2007/067564 |

**C (Continuation). DOCUMENTS CONSIDERED TO BE RELEVANT**

| Category* | Citation of document, with indication, where appropriate, of the relevant passages | Relevant to claim No. |
| --- | --- | --- |
| Y | JP 2-17127 A (Sand AG.),<br>22 January, 1990 (22.01.90),<br>Particularly, Claims; examples<br>(Family: none) | 1-8 |
| Y | JP 2007-9199 A (Maruho Kabushiki Kaisha),<br>18 January, 2007 (18.01.07),<br>Full descriptions<br>(Family: none) | 1-8 |
| Y | JP 2007-45762 A (Shiseido Co., Ltd.),<br>22 February, 2007 (22.02.07),<br>Particularly, Claims; page 7, lines 24 to 36;<br>examples<br>(Family: none) | 1-8 |
| Y | JP 2007-169214 A (Kenji NAKAMURA),<br>05 July, 2007 (05.07.07),<br>Particularly, Claims; page 5, lines 25 to 35;<br>examples<br>& EP 1801184 A1      & US 2007/149624 A1 | 1-8 |
| Y | JP 2003-212716 A (Hifu Rinsho Yakuri Kenkyusho Kabushiki Kaisha),<br>30 July, 2003 (30.07.03),<br>Particularly, Claims; examples; page 14, left column, line 45 to page 15, left column, line 3<br>(Family: none) | 1-8 |
| Y | JP 63-287718 A (Shiseido Co., Ltd.),<br>24 November, 1988 (24.11.88),<br>Full descriptions<br>(Family: none) | 1-8 |

Form PCT/ISA/210 (continuation of second sheet) (April 2007)

<table>
<tr><td>**INTERNATIONAL SEARCH REPORT**</td><td>International application No.<br>PCT/JP2007/067564</td></tr>
</table>

**Box No. II      Observations where certain claims were found unsearchable (Continuation of item 2 of first sheet)**

This international search report has not been established in respect of certain claims under Article 17(2)(a) for the following reasons:

1. ☒ Claims Nos.: 9-17
   because they relate to subject matter not required to be searched by this Authority, namely:

   Claims 9 to 17 pertain to methods for treatment of the human body by therapy and thus relate to a subject matter which this International Searching Authority is not required, under the provisions of the PCT Rule 39.1(iv), to search.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III      Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**    ☐ The additional search fees were accompanied by the applicant's protest and, where applicable,
the    payment of a protest fee.

       ☐ The additional search fees were accompanied by the applicant's protest but the applicable protest fee was not paid within the time limit specified in the invitation.

Form PCT/ISA/210 (continuation of first sheet (2)) (April 2007)

## REFERENCES CITED IN THE DESCRIPTION

**Patent documents cited in the description**

- JP 5310591 A
- WO 9300160 A
- JP 7188046 A
- JP 7278007 A
- WO 9522343 A
- JP 8133979 A
- JP 10007584 A
- EP 0793966 A
- JP 2005516931 W
- WO 03051385 A
- JP 7025784 A

**Non-patent literature cited in the description**

- **Draize, J.H.** Appraisal of the safety of chemicals in foods, drugs and cosmetics. Association of Food and Drug Officials of the United States, 1965, 46-59